# EUROPEAN PATENT APPLICATION

(11) **EP 3 988 008 A1**
(43) Date of publication of application: **27.04.2022**
(21) Application number: 19933346.9
(22) Date of filing: 20.06.2019
(51) Int. Cl.: A61B 1/01

(54) **OVERTUBE ASSEMBLY**

(71) Applicant: The Jikei University, Tokyo 105-8461 (JP); Muranaka Medical Instruments Co. Ltd., Osaka-shi, Osaka 540-0036 (JP); Okumura Co., Ltd., Matsuzaka-shi Mie 515-0841 (JP)
(72) Inventor: SUMIYAMA, Kazuki, Tokyo 105-8461 (JP); KAMBA, Shunsuke, Tokyo 105-8461 (JP); WAKITA, Shoji, Osaka-shi, Osaka 540-0036 (JP); TANAKA, Hiroto, Matsusaka-shi, Mie 515-0841 (JP)
(74) Representative: Dehns
(86) International application number: PCT/JP2019/024544
(87) International publication number: WO 2020/255345

(57) **Abstract**

Provided is an overtube assembly (1) comprising an overtube (2) having an adjustable length. The overtube assembly (1) has the overtube (2) and a mouthpiece (3). The overtube (2) has a plurality of recesses (5) (e.g., grooves) on the outer circumference thereof. The mouthpiece (3) has a locking portion (19) (e.g., a protrusion or ridge) that is locked to the recesses (5). The mouthpiece (3) can move between a locking position at which the locking portion (19) engages with the recess (5) and an unlocking position at which the locking portion (19) does not engage with the recess (5).

## Description

### TECHNICAL FIELD

The present invention relates to an overtube assembly.

### BACKGROUND ART

Conventionally, there has been known an overtube assembly, comprising an overtube into which an endoscope will be inserted, an adaptor mounted on a proximal end of the overtube, and a mouthpiece for positioning the overtube relative to the patient. See, for example, Patent Document 1.

A bellows tube is also known for a medical tube. See, for example, Patent Document 2.

The overtube of Patent Document 1 is made of plastic tube reinforced with a metal coil mounted inside the body of the overtube, so that the overtube is hard to be cut to adjust its length. This means that a number of overtubes with different lengths need to be prepared for patients with different figures.

The medical tube of Patent Document 2 is simply a bellows hose. Further, Patent Document 2 fails to teach or suggest that the bellows hose may be cut to adjust its length.

### PRIOR ART DOCUMENTS

### PATENT DOCUMENTS

Patent Document 1: JP 2008-220794 A
Patent Document 2: JP 2014-113179 A

### SUMMARY OF THE INVENTION

### PROBLEMS TO BE SOLVED BY THE INVENTION

An object of the present invention is to provide an overtube assembly, in which a length of the overtube from the mouthpiece to the distal end of the tube can be adjusted.

### MEANS FOR SOLVING THE PROBLEMS

An overtube assembly (1) according to one embodiment of the present invention comprises:
a flexible hollow cylindrical overtube (2) having openings at opposite ends of the overtube (2); and
a mouthpiece (3) configured to be mounted around an outer circumference of the overtube (2) for movement in a direction along a longitudinal central axis of the overtube (2) ;
wherein the overtube (2) has a plurality of recesses (5) formed on the outer circumference of the overtube (2) at a regular interval in the direction along the longitudinal central axis, and
wherein the mouthpiece (3) has a locking portion (19) configured to engage with the recess (5) of the overtube (2) .

With the overtube assembly (1) so constructed, a length of the overtube (2) from the mouthpiece (3) to the distal end of the overtube (2) can be adjusted by changing the recess (5) with which the locking portion (19) of the mouthpiece (3) engages and thereby the position of the mouthpiece (3) relative to the overtube (2). Accordingly, there is no need to hold a variety of overtubes with different lengths.

### BRIEF DESCRIPTION OF THE DRAWINGS

Fig. 1 is a perspective view of an overtube assembly according to an embodiment of the present invention.
Fig. 2 is an exploded perspective view of the overtube assembly shown in Fig. 1.
Fig. 3 is an enlarged partial cross sectional view of an overtube of the overtube assembly shown in Fig. 1.
Fig. 4 is a partial cutaway perspective view of a mouthpiece of the overtube assembly shown in Fig. 1.
Fig. 5 is a partial cutaway perspective view of an adaptor of the overtube assembly shown in Fig. 1.

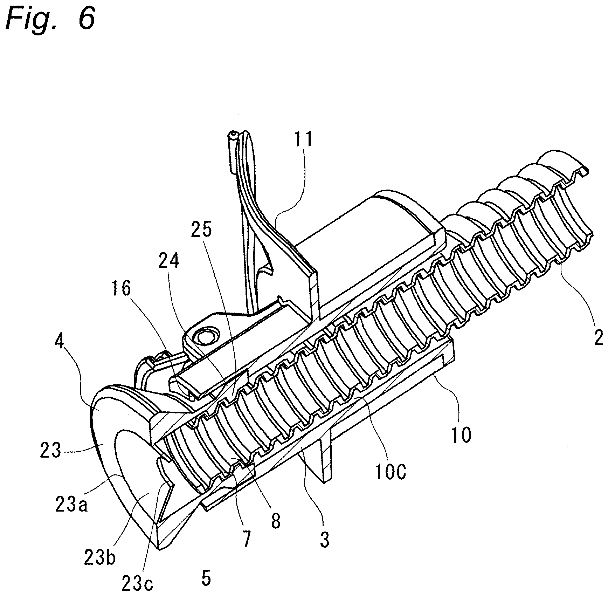

Fig. 6 is a partial cutaway perspective view of the overtube assembly shown in Fig. 1.
Fig. 7 is a partial cutaway perspective view of the overtube assembly shown in Fig. 1.
Fig. 8 is a partial perspective view of the mouthpiece of the overtube assembly in Fig. 1, when viewed from the opposite direction.

### EMBODIMENTS OF THE INVENTION

An embodiment of an overtube assembly according to the present invention will be described with reference to the accompanying drawings. In the following descriptions, one end of the overtube to be inserted into a mouth of patient is referred to as "distal end" and the opposite end of the overtube is referred to as "proximal end". Also, one and the other sides of the overtube which appear on the left and right when viewed in a proximal to distal direction are referred to as "left side" and "right side", respectively.

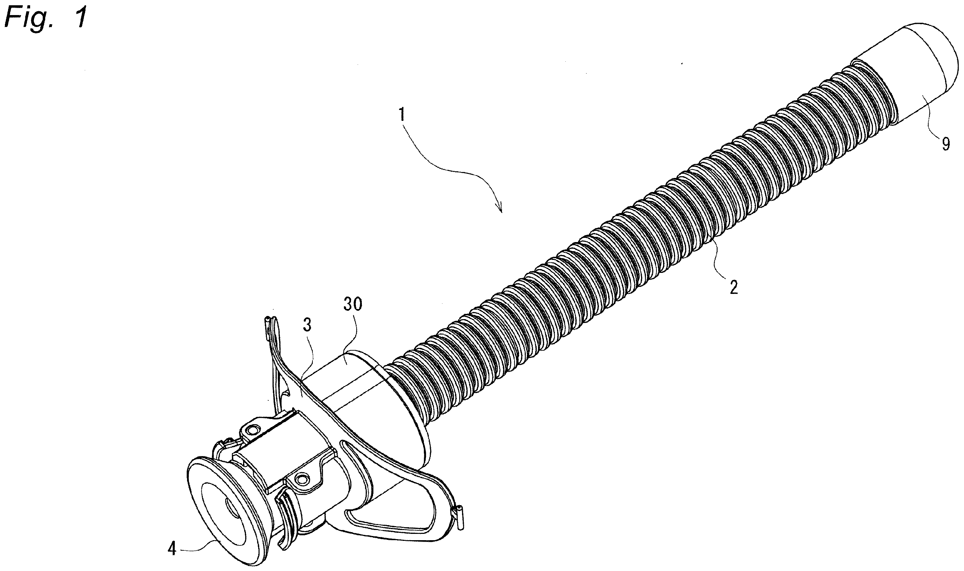

As shown in Fig. 1, the overtube assembly 1 for endoscope according to the embodiment schematically has an overtube 2, a mouthpiece 3, and an adaptor 4.

The overtube 2, which is made by molding a synthetic resin such as polyethylene, is in the shape of hollow cylinder with openings at opposite ends thereof. Structures and thicknesses of respective portions of the overtube are determined such that the overtuce can be deformed easily by an application of small forces.

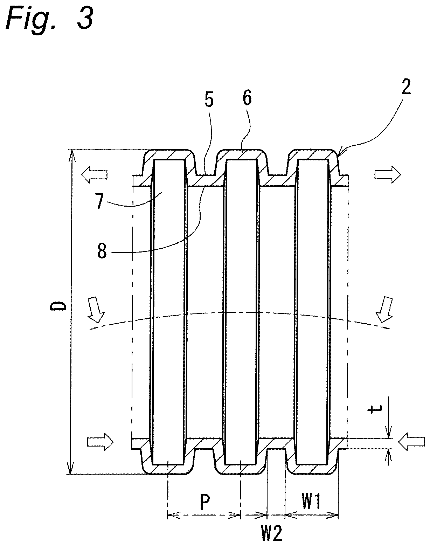

For example, as shown in Fig. 3, the overtube 2 of the embodiment is a flexible tube having small and large diameter portions arranged alternately and continuously in a direction along the longitudinal axis of the overtube. Specifically, the overtube 2 includes, on its outer circumferential surface, outer annular grooves 5 recessed radially inward and outer annular projections 6 each positioned between adjacent outer annular grooves 5 and projected radially outward such that the outer annular grooves 5 and the outer annular projections 6 are arranged alternately in the longitudinal central axis of the overtube 2. As shown, a width of the outer annular grooves 5 (i.e., a distance W between a pair of side walls opposing in the longitudinal direction to form the groove) gradually increases radially outward from the bottom of groove.

The overtube 2 includes, on its inner circumferential surface, inner annular grooves 7 recessed radially outward and inner annular projections 8 each positioned between adjacent inner annular grooves 7 and projected radially outward such that the inner annular grooves 7 and the inner annular projections 8 are formed alternately and at regular intervals in the longitudinal axis direction of the overtube 2.

In the embodiment, the outer annular groove 5 and the inner annular projection 8 oppose each other leaving a constant thickness therebetween and the outer annular projection 6 and the inner annular groove 7 oppose each other leaving a constant thickness therebetween, such that the overtube 2 has a constant thickness (t) over the entire length of the overtube.

Dimensions and thicknesses of the grooves and the projections of the overtube 2 are respectively determined such that the overtube 2 can be deformed easily by an application of small forces that may be applied during the use of the overtube 2, but not collapsing or closing the inner hollow passage of the overtube, i.e., while maintaining the hollow passage inside the overtube in a stable manner. This allows that an endoscope inserted in the overtube from the proximal end of the overtube is moved in a direction toward the distal end of the overtube with small forces. The overtube 2, which is a flexible tube with alternate large and small diameter portions, has a bending stiffness greater than that of tubes without such large and small diameter portions. Then, the flexible tube, even if it has the same thickness as the conventional tube without large and small diameter portions, ensures a stable space for insertion of the endoscope, without collapsing or closing, even when unexpected forces are applied to the overtube during its use, i.e., at the insertion of the endoscope.

For example, the overtube 2 has following dimensions:
Diameter D: 15.0 mm to 20.0 mm;
Thickness t: 0.3 mm to 1.0 mm;
Pitch P of outer projections 6: 2.0 mm to 4.0 mm;
Width W1 of outer projections 6: 1.0 mm to 2.5 mm;
Width W2 of outer grooves 5: 1.0 mm to 2.5 mm; and
Height H of outer projections 6: 1.2 mm to 2.0 mm.

Each of the dimensions described earlier has a higher priority than the dimensions described later in designing the overtube. The stiffness or collapsing resistibility against the bending force depends largely on, among others, the cross sectional diameter D and the thickness t of the tube. Subsequent to the diameter D and the thickness t, the pitch P of the outer annular projection, the width W1 of the outer annular projection, the width W2 of the outer annular groove, and the height H of the outer annular projection affect, in this order, the stiffness and bending resistibility of the tube.

By determining each dimension of the sections of the overtube 2 as described above, the overtube 2 is light-weighted. Further, the alternate and continuous arrangement of the outer annular grooves 5 and projections 6 increases the rigidity of the overtube 2, which allows that the bending of the overtube 2 does not result in any collapsing or closing of the inner passage of the overtube, i.e., the overtube maintains cylindrical passage thereinside. As described above, the overtube 2 is ligh-weight and has a structure capable of being easily bent, which ensures the overtube to be inserted smoothly into the body of the patient, e.g.., through the mouth cavity into the esophagus of the patient.

Preferably, as shown, the circumferentially running corners of the outer annular projections 6 are chamfered and smoothed. This ensures that the overtube 2 is inserted from the mouth into the body of the patient without damaging pharynx or esophagus of the patient.

Although, in the embodiment, the longitudinal cross sections of the outer annular grooves 5 and the inner annular projections 8, i.e., the outer and inner surface configurations, particularly shown in Fig. 3, are in the form of bracket, at least one of the longitudinal cross sections of the outer annular groove 5, the outer annular projection 6, the inner annular groove 7, and the inner annular projection 8 may be circular-, U-, or triangular-shaped.

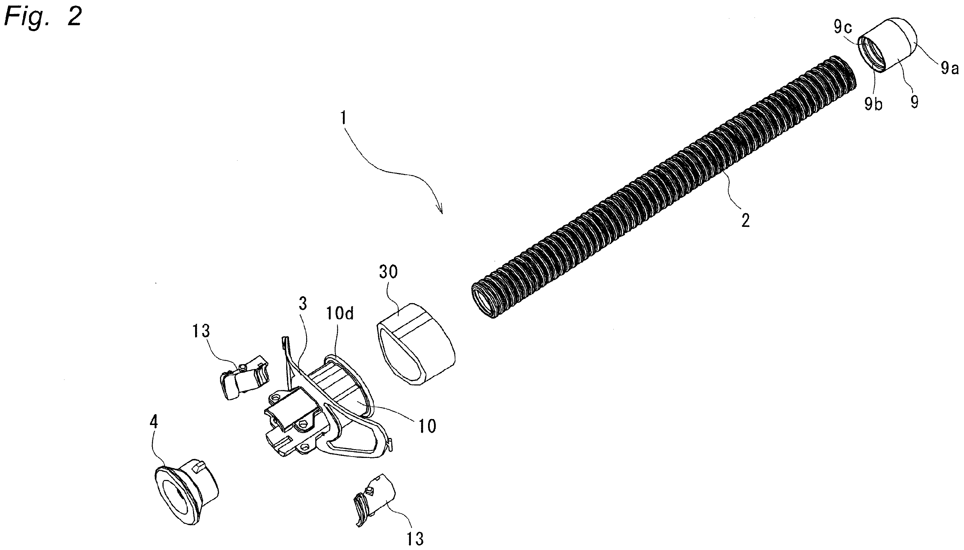

Referring back to Figs. 1 and 2, according to the embodiment, a cylindrical cover 9 is fixed on the distal end of the overtube 2. Preferably, the cover 9 is made of material (for example, silicon rubber) having a softness greater than the overtube 2. Instead, the cover 9 may be made of material other than silicone rubber (for example, a polyvinyl chloride having a hardness greater than the silicone rubber), in which the necessary softness can be obtained by reducing a thickness of the cover.

In the embodiment, as shown in Fig. 2, an outer surface of the distal end portion 9a of the cover 9 is tapered such that the outer diameter of the cover 9 decreases gradually toward the tip of the distal end portion. The inner circumferential surface of the cover 9 is formed with a plurality of alternate small-diameter annular projections 9b and large-diameter annular grooves 9c shaped respectively in correspondence with the outer annular grooves 5 and projections 6 of the overtube 2 such that the cover 9 is secured on the overtube 2 by engaging the small-diameter annular projections 9b and the large-diameter annular grooves 9c of the cover 9 respectively with the outer annular grooves 5 and the outer annular projections 6 of the overtube 2.

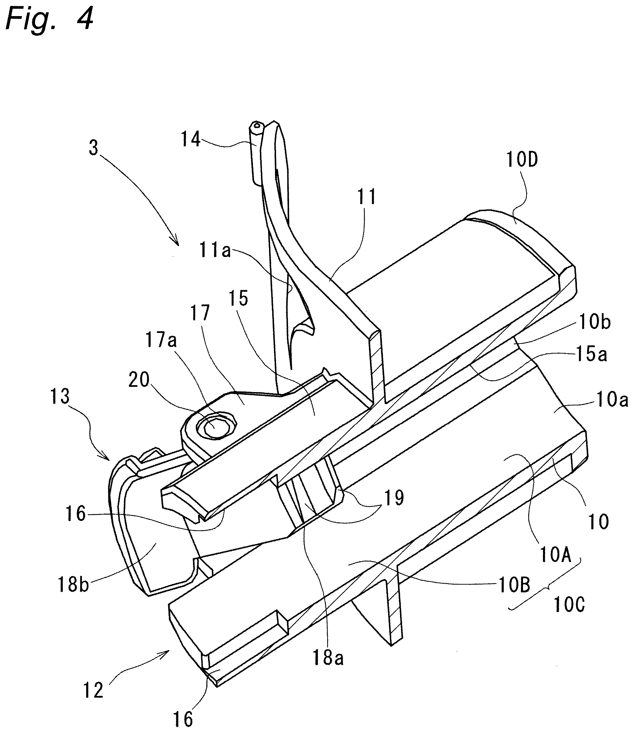

The mouthpiece 3 is made of a synthetic resin (e.g., an acrylonitrile butadiene styrene (ABS) resin) that is greater in hardness than the material of the overtube 2. As shown in Figs. 2 and 4, the mouthpiece 3 has a hollow cylindrical portion 10 to be inserted into the mouth of the patient. The cylindrical portion 10 supports a hollow cylindrical cushioning member 30 mounted around the cylindrical portion 10. The cushioning member 30 is made of a soft material such as a urethane or the silicone rubber. In use, the cushioning member 30 is also inserted into the mouth of the patient as it is retained on the cylindrical portion 10, i.e., without dropping, by the engagement with a flange 10D formed integrally on the distal end portion of the cylindrical portion 10. Due to the interposition of the cushioning member 30, the patient is easily capable of holding the mouthpiece 3 in his or her mouth.

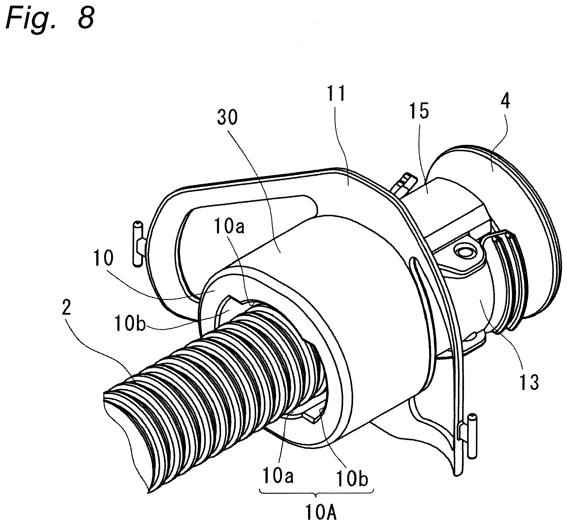

The cylindrical portion 10 has a first cylindrical surface 10A through which the overtube 2 is inserted. A cross section of the first cylindrical surface 10A is not a complete circle and slightly ellipses such that the a length in the left to right direction (major axis) is larger than that in the vertical direction (minor axis). Specifically, as shown in Fig. 8, the first cylindrical surface 10A has upper and lower small diameter arch surface portions 10a and left and right large diameter arch surface portions 10b having larger diameter than the small diameter arch surface portions 10a. The cross section of the cylindrical portion 10 is slightly greater than the cross section of the overtube 2.

A face plate 11 or flange is secured at the proximal end of the cylindrical portion 10. The faceplate 11 extends radially outward from the entire circumference of the proximal end of the cylindrical portion 10. In the embodiment, the faceplate 11 extends in the left to right direction than the upper to lower direction. The left and right flange portions of the face plate 11 are slightly curved distally such that, when the patient holds the cylindrical portion 10 with the cushioning member 30, the left and right flange portions extend on and along the let and right cheeks of the patient. The left and right flange portions of the face plate 11 have respective openings 11a for oral caring and respective hooks 14 formed integrally at the leftward and rightward ends of the flange portions for engagement with fixing strings, for example.

The faceplate 11 has a holder 12 formed integrally on a proximal side surface of the faceplate 11, i.e., a surface opposite the surface where the cylindrical portion 10 is connected. In the embodiment, the holder 12 has a pair of flanges 15 extending proximally from the faceplate 11. In the embodiment, the pair of flanges 15 have upper and lower flanges spaced away from each other in the vertical direction. As shown in Fig. 4, a lower surface of the upper flange (a surface opposing the lower flange) and an upper surface of the lower flange (a surface opposing the upper flange) run on a second cylindrical surface 10B that is assumed by extending the first cylindrical surface 10A proximally. The first cylindrical surface 10A and the second cylindrical surface 10B (specifically, the lower surface portion of the upper flange and the upper surface portion of the lower flange) cooperate with each other to form a cylindrical passage 10C in which the overtube 2 will be inserted.

As shown in Fig. 4, the cylindrical lower surface portion of the upper flange and the cylindrical upper surface portion of the lower flange have, at respective central portions thereof, positioning portions (recessed portions) 16 in the form of grooves running proximally from the proximal end of the upper and lower flanges. Also, the left and right longitudinal edges of the upper flange are extended radially outward to form left and right upper bearing flanges 17, while the left and right longitudinal edges of the lower flange are extended radially outward to form left and right lower bearing flanges 17.

The left upper and lower flanges 17 have a pair of bearing holes 17 formed along a vertical axis (not shown) extending orthogonal to the longitudinal axis of the cylindrical passage 10C, while the right upper and lower flanges 17 have a pair of bearing holes 17 formed along a vertical axis (not shown) extending orthogonal to the longitudinal axis of the cylindrical passage 10C. On each side, a locking plate (rotatable member) 13 is disposed between the upper and lower bearing flanges 17. The locking plate 13 has a distal side portion 18a and a proximal side portion 18b. The distal and proximal side portions 18a and 18b cooperate with the inner surface portions of the upper and lower flanges 15 to form an inner cylindrical surface that is substantially the same as or slightly larger than the inner cylindrical surface of the cylindrical passage 10C. The inner surface of the distal side portion 18a is not aligned with or deflected from the inner surface of the proximal side portion 18b such that the inner surface of the proximal side portion 18b is extended obliquely and outwardly from the proximal end of the inner surface of the distal side portion 18a. The distal side portion 18a of the locking plate 13 has a locking portion 19 (first engagement portion) formed on the inner surface of distal side portion. In the embodiment, the locking portion 19 has a configuration that is substantially the same as the outer annular grooves 5 of the overtube 2. Instead, the locking portion 19 may be a mere projection or projections. As shown in Fig. 4, a plurality of circumferentially extending projections may be formed on each locking plate 13. Preferably, a pitch of the projections in the longitudinal direction is set to be the same as or integer multiple of the pitch of the outer annular grooves 5 of the overtube 2.

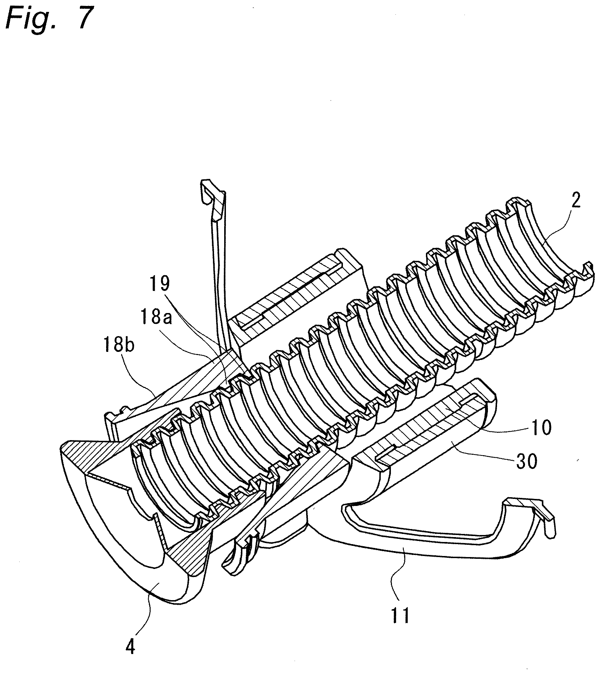

The distal side portion 18a and the proximal side portion 18b respectively have heights or vertical length substantially the same as the distance between the upper and lower bearing flanges 17. Upper and lower longitudinal edge portions in the vicinity of the boundary of the distal side portion 18a and the proximal side portion 18b have bearing portions or shafts 20 formed integrally therewith and projecting upward and downward, respectively. This allows that the left and right locking plates 13 are disposed between the upper and lower bearing flanges 17 with the upper and lower shafts 20 inserted in the upper and lower bearing holes 17a such that the locking plate 17 can pivot between a locking position (shown in Figs. 4 and 7) in which the locking portions 19 project inside the second cylindrical surface 10B and an unlocking position (not shown) in which the locking portions 19 stay outside the second cylindrical surface 10B.

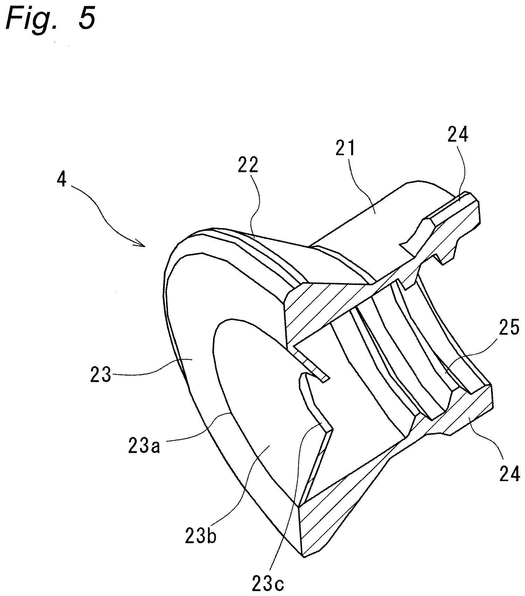

As shown in Fig. 1, the adaptor 4 is removably mounted on the proximal end of the overtube 2. As shown in Fig. 5, the adaptor 4 is a cylindrical member made of relatively soft synthetic resin material such as silicone rubber. In the embodiment, the adaptor 4 has a cylindrical portion formed at the distal end thereof. The cylindrical portion 21 has an inner diameter substantially the same as the outer diameter of the overtube 2 and an outer diameter substantially the same as the inner diameter of the proximal second cylindrical surface 10B of the mouthpiece 3.

The cylindrical portion 21 has upper and lower positioning portions (projections) 24 projecting radially upward and downward, respectively, formed on the upper and lower circumferential surface portions of the cylindrical portion 21. The positioning portions 24 correspond to the positioning portions (recesses) 16 formed in the upper and lower flanges 15, respectively.

The cylindrical portion 21 has an engagement portion (second engagement portion) 25 formed integrally on the inner surface of the cylindrical portion 21 and projected radially inward therefrom. In the embodiment, the engagement portion 25 is made of one or more elongate ridges (two elongate ridges in the embodiment) having a configuration corresponding substantially to the outer annular grooves 5 of the overtube 2. Instead, the engagement portion 25 may be made of one or more small projections.

The cylindrical portion 21 connects to a conical cylindrical portion 22. The inner (outer) diameter of the conical cylindrical portion 22 increases gradually from the proximal (distal) end to the distal (proximal) end of the conical cylindrical portion. The distal (proximal) end of the conical cylindrical portion 22 connects to an annular portion 23. The annular portion 23 extends radially inward from the distal (proximal) end to form a circular central opening 23a. An annular valve plate 23b is formed to extend radially inward from the opening 23a such that the diameter gradually decreases proximally (distally). The annular valve plate 23b has a small thickness such that it is easy to deform. A diameter of the central opening 23c defined inside the annular valve plate 23b is determined to be smaller than the outer diameter of inserting members. This allows that the inner circumferential edge defining the central opening 23c makes a sealing contact on the outer circumferential surface of the inserting member inserted through the central opening 23c into the adaptor 4.

When assembling the overtube assembly 1 with the mouthpiece 3, the overtube 2 with the cover 9 mounted on the distal end thereof is inserted through the distal or proximal end opening into the cylindrical passage 10C of the mouthpiece 3. In this instance, the left and right locking plate 13 stays in the unlocking position to avoid an interference between the overtube 2 and the locking portion (first locking portion) 19, which would otherwise be caused at the insertion of the overtube into the cylindrical passage 10C in the distal to proximal direction or in the opposite direction.

When adjusting the length of the overtube 2 to match with the figure of the patient, a proximal end portion of the overtube adjacent the mouthpiece 3 is cut off as necessary. The thickness of the overtube 2 is relative small and the endless outer annular groove 5 continues annularly, which ensures a smooth and clear cut edge. Also, the outer annular grooves 5 have a constant pitch, such that the cutting position is easy to recognize. For example, for the overtube in which the outer annular grooves 5 are arranged at regular pitches of 3 mm, when cutting off by 3.0 cm, the overtube is cut at approximately tenth groove from the proximal end without any need to measure the distance from the distal end by using scale.

After adjusting the length of the overtube 2, the adaptor 4 is fitted on the proximal end of the overtube 2, allowing the inner engagement portion (second engagement portion) 25 of the adaptor to engages in the outer annular grooves 5 of the overtube, which results in that the adaptor 4 is removably fixed to the overtube 2.

In use, i.e., in insertion of the overtube, an endscope is inserted through the overtube 2. Then, the endscope is inserted into the esophagus of the patient, followed by inserting the overtube 2 from the mouth through the pharynx into the esophagus as it is guide by the endoscope. The overtube 2, as it is flexible due to the alternate and continuous outer and inner annular grooves 5 and 7, is bent or curved easily along the pharynx and then the esophagus of the patient. Also, the annular walls of the overtube ensures a stable open passage inside the overtube without causing a local reduction of the cross section even if a radiallly inward pressure is applied locally to the overtube. The inserting portion 10 is inserted in the mouth of the patient. Then, the strings on the left and right hooks 14 are tied with each other on the back of the head of the patient to hold the mouthpiece 3 on the patient.

Once the distal end of the overtube 2 reaches the target position, the distal side portion of the left and right locking plates 13 are forced to move toward each other, moving the locking plates 13 from the unlocking position to the locking position, which causes that the locking portions 19 engages in the outer annular grooves 5 of the overtube 2 and thereby the overtube 2 is held by the mouthpiece 2.

Also, once the distal end of the overtube 2 reaches the target position, the positioning portion (projected portion) 24 of the adaptor 4 engages with the associated positioning portion (recessed portion) 16, preventing a rotation of the overtube 2, relative to the mouthpiece 3, around the longitudinal central axis of the overtube.

In this condition, insertions and extractions of the endscope may be carried out through the central opening 23c of the adaptor 4 and the inner passage of the overtube 2 for necessary procedures.

After completion of the preocedures, in order to remove the inserted overtube 2 from the patient, the proximal side portion of the left and right locking plates are forced to each other to move the locking plates 13 from the locking position to the unlocking position, which allows the overtube 2 to be pulled out from the patient.

The overtube assembly described above may be modified in various ways.

For example, the grooves on the outer surface of the overtube 2 are not necessarily the endless annular grooves, they may be one or more spiral grooves instead.

Also, the first locking portion 19 may be provided at the proximal end portion 18b instead of at the distal end portion 18a of each of the locking plates 13.

Further, although in the embodiments described above the locking plates 13 are provided on the left and right sides of the mouthpiece 3, at least one locking plate may be provided on the left or right side. According to the embodiment described above, the locking plates on opposite sides allows users to press them by, for example, a thumb and an index finger, which ensures a stable locking (unlocking) operation.

### PARTS LIST

- 1: overtube assembly
- 2: overtube
- 3: mouthpiece
- 4: adaptor
- 5: outer annular groove (recess)
- 9: cover
- 10: cylindrical portion
- 10C: cylindrical passage
- 11: faceplate (flange)
- 12: holder
- 13: locking plate (pivot member)
- 18a: distal end portion
- 18b: proximal end portion
- 19: locking portion

## Claims

1. An overtube assembly (1) comprising:
a flexible hollow cylindrical overtube (2) having openings at opposite ends of the overtube (2); and
a mouthpiece (3) configured to be mounted around an outer circumference of the overtube (2) for movement in a direction along a longitudinal central axis of the overtube (2) ;
wherein the overtube (2) has a plurality of recesses (5) formed on the outer circumference of the overtube (2) at a regular interval in the direction along the longitudinal central axis, and
wherein the mouthpiece (3) has a locking portion (19) configured to engage with the recess (5) of the overtube (2) .

2. The overtube assembly (1) according to claim 1, wherein the locking portion (19) is configured to be moved between a locking position in which the locking portion (19) is in engagement with the recess (5) and an unlocking position in which the locking portion (19) is out of engagement with the recess (5).

3. The overtube assembly (1) according to claim 1,
wherein the mouthpiece (3) has a cylindrical passage (10C) through which the overtube (2) is to be inserted, and
wherein the locking portion (19) is configured to be moved between a locking position in which the locking portion (19) protrudes in the cylindrical passage (10C) and engages with the recess (5) and an unlocking position in which the locking portion (19) stays outside the cylindrical passage (10C) and disengages with the recess (5) .

4. The overtube assembly (1) according to claim 3,
wherein the mouthpiece (3) has a pivoting member (13) configured to be pivoted about a shaft (20) disposed orthogonal to the longitudinal central axis of the cylindrical passage (10C),
wherein the pivoting member (13) has a distal side portion (18a) positioned on a distal side with respect to the shaft (20) and a proximal side portion (18b) positioned on a proximal side with respect to the shaft (20), and
wherein the locking portion (19) is provided on either or both of the distal side portion (18a) and the proximal side portion (18b).

5. The overtube assembly (1) according to claim 4, wherein the pivoting member (13) is disposed on either side of the cylindrical passage (10C) to oppose each other across the cylindrical passage (10C).

6. The overtube assembly (1) according to any one of claims 1 to 5, wherein the recesses (5) are grooves running in a circumferential direction of the overtube (2).

7. The overtube assembly (1) according to claim 6, wherein each of the grooves is an endless annular groove running continuously in the circumferential direction of the overtube (2).

8. The overtube assembly (1) according to claim 6, wherein the grooves are portions of a spiral groove runnig spirally in the circumferential direction of the overtube (2) .

9. The overtube assembly (1) according to any one of claims 6 to 8, wherein the locking portion (19) is an elongated ridge formed in correspondence with the grooves.
